# EUROPEAN PATENT APPLICATION

(11) **EP 4 822 336 A1**
(43) Date of publication of application: **14.10.2026**
(21) Application number: 25169319.8
(22) Date of filing: 09.04.2025
(51) Int. Cl.: G01N 21/03, G01N 21/3504, G01N 21/359, G01N 21/39, G01N 21/552, G01N 33/28

(54) **STATIC ELECTRIC INDUCTION DEVICE AND OPERATION METHOD**

(71) Applicant: Hitachi Energy Ltd, 8050 Zürich (CH)
(72) Inventor: Buijs, Henry L., Quebec, G3E 1H7 (CA)
(74) Representative: Epping - Hermann - Fischer

(57) **Abstract**

In at least one embodiment, the static electric induction device (1) comprises:
- a tank (2) configured to be filled with an electrically insulating liquid (3), and
- a gas analyzer (5) configured to provide concentration data indicative of an oxygen concentration of a gas sample (40), wherein
- the gas analyzer (5) comprises a laser (51) configured to emit a test radiation (52) which is narrow-band radiation, and
- the concentration data is optical absorption data of the test radiation (52) through the gas sample (40).

## Description

A static electric induction device is provided. Further, an operation method for such a static electric induction device is also provided.

Document US 7 811 362 B2 discloses an oil/gas separation membrane.

Document US 4 293 399 A refers to detecting and measuring the concentration of gaseous hydrogen dissolved in a fluid.

Documents US 5 047 639 A and US 5 491 341 A disclose laser-based concentration detectors.

Document US 2012/0290229 A1 refers to a diagnostic method for assessing the insulation condition of electrical equipment insulated with oil.

Document US 5 570 697 A refers to an on-airway breath-by-breath oxygen sensor.

A problem to be solved is to provide a static electric induction device in which an oxygen content in an electrically insulating liquid can reliably and efficiently be determined.

This object is achieved, inter alia, by a static electric induction device and by an operation method as defined in the independent claims. Exemplary further developments constitute the subject-matter of the dependent claims.

For example, the static electric induction device is a transformer device wherein an oil-tight but gas-permeable membrane is next to tank to be filled with a transformer oil, and the gas obtained by means of the gas-permeable membrane is put into a cell through which a narrow-line laser is led. Said laser may have a very high power density per nanometer to overcome the well-known very weak absorptivity of the oxygen absorption spectrum and may be tuned narrowly across an oxygen, O₂, absorption line at about 761 nm and thereby provides a reliable concentration of oxygen independent of the possible presence of as many as eight other evolved gases all having a much higher absorptivity, as much as a factor of 100,000, than oxygen. Also, the weakness of absorptivity of oxygen could not be offset by extracting a large volume of gas which could affect the viability of the liquid oil volume and time required to collect it. By measuring the absorption at said oxygen absorption line, a concentration of the oxygen in the obtained gas and, thus, in the transformer oil can be determined. By doing so it can be controlled, for example, if there is a leakage in the tank so that a protective gas or the transformer oil itself came into contact with air.

According to at least one embodiment, the static electric induction device is a power electronic device configured to handle currents of at least 0.1 kA and/or voltages of at least 3.6 kV. For example, the static electric induction device is a transformer device including a transformer. Alternatively or additionally, the static electric induction device is or comprises an electric shunt reactor, an electric series reactor, like a current limiting reactor, an instrument transformer, a capacitor, a disconnector, an air-insulated switchgear, AIS, a gas insulated switchgear, GIS, and/or a gas insulated transmission line, GIL.

According to at least one embodiment, the static electric induction device comprises a tank. The tank is configured to be filled partially or completely with an electrically insulating liquid, like a transformer oil. It is possible that the tank is airtight. That is, in the intended use of the tank there is no or no significant exchange of a gas between an interior and an exterior of the tank. The tank may be based on steel. It is further possible that the tank is configured to be filled with the electrically insulating liquid and to be complemented with a protective gas, like dried N₂ or Ar. Thus, in intended use the tank may completely be filled with the electrically insulating liquid and the protective gas.

According to at least one embodiment, the static electric induction device comprises a gas extractor. The gas extractor is configured to extract a gas sample from the electrically insulating liquid. For example, the gas extractor comprises or is the semi-permeable membrane through which gas can diffuse out of the electrically insulating liquid into a test volume free of the electrically insulating liquid.

According to at least one embodiment, the static electric induction device comprises a gas analyzer. The gas analyzer is configured to receive the gas sample from the gas extractor and is further configured to provide concentration data indicative of an oxygen concentration of the gas sample. In this context, the term 'oxygen' refers to O₂. It is possible that the gas analyzer is configured to be sensitive for O₂ only.

Said gas analyzer sensitive for O₂ only may be supplemented by additional gas analyzers or gas sensors configured to be sensitive for carbon compounds or hydrogen or the like.

According to at least one embodiment, the gas analyzer comprises one or a plurality of lasers. The at least one laser is configured to emit a test radiation. For example, the test radiation consists of a single longitudinal mode which possibly limits a line width at half maximum of the laser to at most 7 x 10⁻⁵ nm or to at most 4 x 10⁻⁵ nm.

For example, the test radiation is narrow-band radiation. This means, for example, that the test radiation has a spectral full width at half maximum, FWHM, of at most 0.1 nm or of at most 0.01 nm or of at most 0.001 nm. Said value may be at least 10⁻⁶ nm or at least 10⁻⁵ nm, like 10⁻⁴ nm. Alternatively or additionally, a relative line width is at most 10⁻⁵ or is at most 10⁻⁶ and/or is at least 10⁻¹⁰ or is at least 10⁻⁸ wherein the relative line width is the spectral FWHM divided by the wavelength of maximum intensity of the test radiation.

According to at least one embodiment, the at least one laser is configured for single mode emission of the test radiation. Thus, narrow-band emission and high spectral power density can be ensured.

According to at least one embodiment, the concentration data is optical absorption data of the test radiation through the gas sample. In other words, a change of transmission of the test radiation caused by the oxygen in the gas sample is determined as, neglecting any reflectivity of the gas sample, the transmission plus the absorption amounts to 1.

In at least one embodiment, the static electric induction device comprises
- a tank configured to be filled with an electrically insulating liquid, and
- a gas analyzer configured to provide concentration data indicative of an oxygen concentration of a gas sample obtained from the electrically insulating liquid,
wherein
- the gas analyzer comprises a laser configured to emit a test radiation which is narrow-band radiation, and
- the concentration data is optical absorption data of the test radiation through the gas sample.

For example, the static electric induction device comprises
- a tank configured to be filled with an electrically insulating liquid,
- a gas extractor configured to extract a gas sample from the electrically insulating liquid, and
- a gas analyzer configured to receive the gas sample from the gas extractor and to provide concentration data indicative of an oxygen concentration of the gas sample, wherein
- the gas analyzer comprises a laser configured to emit a test radiation which is narrow-band radiation, and
- the concentration data is optical absorption data of the test radiation through the gas sample.

Thus, for example, a Tunable Diode laser, TDL, based oxygen analyzer for dissolved gases in transformer oil is provided.

It is possible to determine the composition of dissolved gases in, for example, transformer oil for the purpose of determining the health of a transformer device. Determination of oxygen is of interest to monitor for air leaks in hermetically sealed transformer devices and other possible mechanisms of oxygen generation in transformer oil. For this purpose, a small quantity of dissolved gases are extracted from transformer oil via a permeable membrane that transmits the gases and blocks the liquid phase oil. Possible methods of analysis are infrared absorption spectroscopy, infrared photo-acoustic detection and gas chromatography methods.

Extraction of gases from the liquid oil is a slow process producing only small amounts of gas sample in a given time. Therefore, the amount of gas sample required for analysis greatly influences the response time of the analysis. Hence, transformer oil monitoring systems are to be designed to require small amounts of sample for analysis.

Being a homo-nuclear diatomic molecule, oxygen does not possess a significant infrared signature. It is therefore not easily observed by infrared spectroscopic techniques. An oxygen analyzer may instead use a chemical reaction to determine the amount of oxygen in a sample. However, the consumption of the chemicals require replenishment at regular intervals. The chemical reaction sensors also consume the oxygen present in the sample and therefore force the oxygen analysis to be performed in a so-called batch mode requiring renewal of the sample after each analysis. This is a slower analysis process than the continuous monitoring of the oxygen concentration of a sample in equilibrium with the oxygen dissolved in the oil. The first indication of a fault resulting in a rise in oxygen concentration is the time to change the sample concentration through the membrane and the analysis time to determine the minimum detectable change in oxygen concentration.

Locations of transformer devices can be widely scattered over geographic locations. This is driven by the diversity of electrical power generation and distribution locations. Transformer monitoring using analysis methods that require consumables to be replenished such as gas chromatograph carrier gases and in the case of oxygen, chemical reaction pucks, are undesirable and add a significant cost of ownership over time.

For example, when using zirconium oxide-based sensors to measure oxygen in transformer oil evolved gas, there are a number of issues with this approach which are eliminated with the optical approach described herein. Using such chemical sensors provides limited precision of measurement due to possibilities of cross contamination by other gases, and such sensors have a limited service lifetime and require regular replacement, and their response is quite slow and finally they consume the oxygen they measure causing an unbalance in the equilibrium gas concentrations.

A TDL based oxygen analyzer possessing a long maintenance free life can significantly reduce the long-term cost of ownership when oxygen determination is required. The telecommunication industry has demonstrated that tunable diode lasers, TDLs, are highly reliable devises providing operational lifetimes well in excess of 20 years. Moreover, no interfering effects by any of the numerous gases that may be present in the gas sample are known that may hamper reliability of the optical measurement described herein. This permits good maintenance of accuracy of measurement. No consumption of oxygen occurs in the measurement process, fast measurement, high precision, no drift, no maintenance requirements and no wear out can be achieved, and lasers can have an excellent long service life.

Even though oxygen does not possess a pronounced infrared spectrum, there is an exception to this in the case of simultaneous electronic and rovibrational energy transitions in the molecule. It has been known for many years from the study of solar radiation that oxygen shows several absorption bands in the near infrared that are nevertheless many orders of magnitude weaker than the usual allowed infrared transitions. The spectrum is well known because of absorption by sunlight through many kilometers of atmosphere. However, a sample of oxygen at atmospheric pressure and covering just a 1 m path length shows transmission loss, that is, absorption, of about 10⁻⁴ % in the 760 nm wavelength region. Thus, traditional absorption spectroscopy techniques may not be sensitive enough to be useful for measuring oxygen in a small sample of gas in an efficient manner.

TDLs emit intense light within, for example, a single longitudinal mode which corresponds to a very narrow wavelength interval. The total radiated power is typically between 1 mW and 10 mW, for example. This does not seem much, but when concentrated in a single mode it corresponds to about 175 mW/nm of luminous density which is orders of magnitude higher than any conventional infrared or visible source used in traditional spectroscopy. Additionally, the TDL highly concentrates its light intensity in a small fraction of 1 nm which greatly reduces photon noise. There is enough luminous density to easily measure the very weak absorptions of even small amounts of oxygen in the light beam. Please reconcile with earlier text.

To identify that oxygen is being measured, a minimum spectral interval needs to be examined. This requires tuning the laser wavelength. The structure of the TDL is such that the wavelength has a small dependence on temperature as well as electrical drive current. Since the response to drive current is much faster than to change in temperature, the temperature can be kept constant while the current is varied in order to scan the wavelength through the profile of one of the oxygen spectral lines.

For example, the gas analyzer described herein may fulfil the following requirements, individually or in any combination:
- Small sampling volume: Gas extraction from the oil using a permeable membrane is a slow process. As the volume of gas required for sampling increases, the response time of the analysis increases. A response time, T90, of one hour or less is commonly required.
- Sensitivity: The sensitivity requirement for oxygen is less demanding than for other gases like H₂, CH₄ and/or C₂H₂. A minimum detection limit of 200 ppm oxygen may be acceptable.
- Concentration range: 200 ppm up to 200.000 ppm as in air.
- Operation: Autonomous operation requiring a single voltage electrical power, data availability for oxygen concentration and fault alarm and diagnostic reporting.
- Calibration: Air sample, at atmospheric or other known pressure.
- Environmental: Laser temperature is controlled by a thermo-electric controller, TEC, for example, to +/-0.01 °C of setpoint. A sample cell temperature may be controlled high enough to avoid condensation of any component in the sample.
- Service interval: Design for at least 20 years of zero maintenance operation.

According to at least one embodiment, the test radiation has a wavelength of maximum intensity of at least 760 nm and/or of at most 765 nm. Thus, any number of the numerous oxygen absorption lines in said spectral range can efficiently be observed.

According to at least one embodiment, the laser is configured for a spectral power density of at least 0.27 kW/nm or of at least 0.8 kW/nm. Alternatively or additionally, said value is at most 20 kW/nm or is at most 5 kW/nm. For example, said value is between 1.0 kW/nm and 2.0 kW/nm.

According to at least one embodiment, the gas analyzer further comprises a test cavity. The test cavity is configured to be filled with the gas sample, for example, by the gas extractor or by a part of the gas extractor.

It is possible that the test cavity is a transmission cell for the test radiation. That is, the transmission cell is configured to be passed by the test radiation. For example, the test cavity is configured so that side walls of the test cavity are intentionally not hit by the test radiation. The test cavity may be configured to avoid scattering of the test radiation within the test cavity.

According to at least one embodiment, the test cavity has a radiation entrance window for the test radiation and/or has a radiation exit window different from the radiation entrance window and also for the test radiation. For example, the radiation entrance window and/or the radiation exit window are provided with an anti-reflection coating for the test radiation.

According to at least one embodiment, in the test cavity the test radiation travels a same section in a same direction only once. In other words, the test cavity is then not a resonator in which the test radiation would travel a same section multiple times in both forward and backward directions in order to increase the pathlength and thereby the sensitivity. Not using an optical resonator simplifies aligning the gas analyzer.

According to at least one embodiment, the test cavity is a multi-pass transmission cell. Thus, the test radiation may pass through the transmission cell multiple times. The multiple passes are preferably along different sections so that the transmission cell is not an optical resonator.

According to at least one embodiment, the test cavity is of a White cell-type or of a Herriott cell-type, however, the test cavity could also be of another type of a multi-traverse absorption cell, like a circular multi-pass cell or also like a Pfund cell.

According to at least one embodiment, the test cavity is a hollow-core glass fiber. Thus, the test radiation may be led through the glass fiber and the gas sample can be filled in the at least one hollow space in said glass fiber. It is possible that the glass fiber is configured to be provided with the gas sample during operation so that the gas sample may be circulated or led through the glass fiber.

According to at least one embodiment, the gas analyzer further comprises an analyzer housing. For example, the analyzer housing is a metallic or plastic housing in which all other components of the gas analyzer are placed whereas plug sockets or the like may protrude from the analyzer housing. Moreover, the analyzer housing may be provided with supply pipes or offtake pipes with regard to the gas sample to be tested, for example.

According to at least one embodiment, the laser is placed within the analyzer housing. The gas extractor may be placed outside the analyzer housing. For example, the gas extractor is configured to transport the gas sample into the analyzer housing, especially to or into the test cavity. It is possible that within the analyzer housing the gas sample is limited to the test cavity so that a remainder of the analyzer housing may be kept clean from the gas sample. By doing so, an accurate reference measurement may be ensured.

According to at least one embodiment, the test cavity is located completely or partially in the analyzer housing. Thus, the test cavity may be protected from mechanical or chemical damage by means of the analyzer housing, and dirt may be kept away from optical components, and defined temperature conditions may be kept within the analyzer housing.

According to at least one embodiment, the test radiation travels through a housing atmosphere before and/or after entering the test cavity. For example, the housing atmosphere comprises oxygen. Especially, the housing atmosphere could be air or dried air and, thus, may consist of water vapor, about 1% of Ar, 21% of O₂ and 78% of N₂; other gases, like CO₂, may have an overall proportion of at most 0.1% or of at most 0.05%, for example; the term 'dried' may refer to a dew point of at most -20 °C or of at most -40 °C or of at most -60 °C, for example. A protective gas, like dried N₂, Ar or He, may alternatively be used. It is possible that the analyzer housing is once filled with the housing atmosphere and is configured to keep the housing atmosphere. That is, intentionally the housing atmosphere does not vary or does not vary much over time. Hence, defined conditions for a reference measurement may be ensured. Otherwise, the analyzer housing may be configured to have a changing or changeable housing atmosphere, for example, by having the analyzer housing flushed with air from the environment. A pressure of the housing atmosphere is, for example, 1 bar. For example, the pressure of the housing atmosphere is measured to confirm the oxygen concentration in the housing atmosphere especially in case of using air or dried air.

According to at least one embodiment, the gas analyzer further comprises a first radiation detector and a second radiation detector. The first and/or second radiation detector may be located in the analyzer housing. It is possible that there are more than two radiation detectors. Said radiation detectors are configured for detecting the test radiation and, thus, may be silicon-based detectors, like photo diodes, PIN diodes or avalanche diodes.

According to at least one embodiment, path lengths of the test radiation outside the test cavity from the laser to the first radiation detector and to the second radiation detector are the same. By doing so, oxygen in the housing atmosphere may be compensated for a both a measurement radiation measured at the second radiation detector and a reference radiation measured at the first radiation detector may travel through a same portion of oxygen-containing atmosphere. This can permit calibrating the gas analyzer by using a zero signal for a sample detector and determining the atmospheric oxygen in a reference channel only.

According to at least one embodiment, the gas analyzer comprises a cladding-free optical fiber configured to be in direct contact with the gas sample. In this case, the test radiation may be configured to be led through the cladding-free optical fiber so that an evanescent field of the test radiation reaches into the gas sample. In this case, the test radiation may be free of any free-beam sections so that the test radiation may be led exclusively through solid materials. This may increase robustness and may decrease adjustment efforts.

According to at least one embodiment, the electrically insulating liquid is a transformer oil. Optical properties of the transformer oil may not be important as the test radiation may not come into contact with the transformer oil. Thus, the transformer oil may be optically transparent or also opaque.

According to at least one embodiment, the gas analyzer is placed in a test space, especially directly in a test space. The test space is configured to directly adjoin the electrically insulating liquid. In other words, there is no barrier or membrane or intermediate layer between the electrically insulating liquid and the test space. Thus, the test space may be referred to as a head space of the tank. The test space itself is free of the electrically insulating liquid.

According to at least one embodiment, the test space is configured to be filled with the gas sample. This is, for example, due to diffusion of gases out of the electrically insulating liquid into the test space.

According to at least one embodiment, the concentration data is the optical absorption data of the test radiation through the test space. In other words, the laser may directly emit the test radiation into the test space so that the test radiation travels through part of the test space. The at least one radiation detector may directly be placed in the test space or may directly adjoin the test space. Thus, there may be no test cavity and no gas extractor, but the gas analyzer could directly work in the test space.

According to at least one embodiment, the static electric induction device is a transformer device configured for an electric power of at least 0.1 MVA or of at least 1 MVA or of at least 10 MVA. Alternatively or additionally, the transformer device configured for an electric power of at most 1 GVA or of at most 200 MVA. Thus, the static electric induction device may be quite expensive and could be configured for large electric powers requiring efficient cooling by means of the electrically insulating liquid and requiring reliable early detection means for possible damages.

According to at least one embodiment, the gas extractor comprises a separation membrane configured to be in direct contact with the electrically insulating liquid and being impermeable for the electrically insulating liquid. It is possible that the separation membrane is permeable for gases and is configured to be adjacent to a test volume on a side remote from the electrically insulating liquid. The test volume could be configured to be filled with an extracted gas from the electrically insulating liquid, for example, in a diffusion equilibrium with the electrically insulating liquid. Thus, the gas sample may efficiently and reliably be obtained from the electrically insulating liquid.

According to at least one embodiment, the gas extractor further comprises one or a plurality of circulation units. The at least one circulation unit is configured to circulate the extracted gas through the test volume. By said circulation, the test cavity may be provided with the gas sample. For example, the circulation unit is continually circulating the evolved gases in a sealed circuit so that there is no demand for a gas transfer as long as there is no change in concentrations.

According to at least one embodiment, the laser is configured to vary an output wavelength by at least 0.1 nm or by at least 0.5 nm. Alternatively or additionally, said value is at most 5 nm or is at most 2 nm. For example, said value is large enough to completely scan across the oxygen absorption line around 760.8 nm. For example, tunability is about 2 nm by temperature tuning, and a fraction of 1 nm by current tuning.

According to at least one embodiment, the laser is temperature stabilized. A stabilization accuracy is, for example, 0.1 °C or better or is 0.01 °C or better. A temperature at which the laser is stabilized is, for example, at least 250 K or at least 270 K or at least 290 K or at least 305 K. Alternatively or additionally, said value could be at most 340 K or at most 310 K or at most 295 K. Having values of, for example, above 290 K may ensure avoiding a water film next to the laser. For example, the laser has a built-in thermo-electric cooling unit attached to a laser diode chip with a temperature stability of better than +/-0.01 °C and a tuning temperature range of, for example, 20 °C to 80 °C. Current tuning is, for example, +/- 0.1 mA which can be sufficient to scan more than a complete oxygen spectral line.

According to at least one embodiment, the static electric induction device comprises one or a plurality of evaluation units. The at least one evaluation unit is configured to evaluate the concentration data indicative of the oxygen concentration and to output a signal indicative of the oxygen concentration. Thus, overall signal evaluation and, thus, determination of the oxygen concentration may be done inside the static electric induction device, especially inside the gas analyzer. Said signal may be output by using wire-bound or wireless communication means. Preferably, said signal is a digital signal.

According to at least one embodiment, for providing the concentration data indicative of the oxygen concentration, the gas analyzer is free of an optical resonator and is free of a gas chromatography stage. Thus, the concentration data indicative of the oxygen concentration may only be absorption or transmission data of the test radiation through the gas sample.

A method for operating the static electric induction device is additionally provided. For example, by means of the method a static electric induction device as indicated in connection with at least one of the above-stated embodiments is operated. Features of the static electric induction device are therefore also disclosed for the method and vice versa.

In at least one embodiment, the method is for operating a static electric induction device and comprises:
- filling the tank with the electrically insulating liquid,
- operating the static electric induction device,
- extracting, possibly by the gas extractor, the gas sample from the electrically insulating liquid and optionally transporting the gas sample to the gas analyzer,
- transmitting, by the laser, the test radiation through the gas sample, and
- providing, by the gas analyzer, the concentration data indicative of the oxygen concentration of the gas sample.

A static electric induction device and an operation method described herein are explained in greater detail below by way of exemplary embodiments with reference to the drawings. Elements which are the same in the individual figures are indicated with the same reference numerals. The relationships between the elements are not shown to scale, however, but rather individual elements may be shown exaggeratedly large to assist in understanding.

In the figures:
- Figure 1: is a schematic sectional view of an exemplary embodiment of a static electric induction device described herein,
- Figure 2: is a schematic top view of an exemplary embodiment of a gas analyzer for static electric induction devices described herein,
- Figure 3: is a schematic sectional view of an exemplary embodiment of a test cavity for gas analyzers described herein,
- Figure 4: is a schematic sectional view of an exemplary embodiment of a gas extractor for static electric induction devices described herein,
- Figures 5: and 6 are schematic representations of optical transmission data of oxygen in the near-infrared spectral range,
- Figures 7: and 8 are schematic representations of measurement data of an exemplary embodiment of a static electric induction device described herein,
- Figures 9: to 11 are schematic sectional views of exemplary embodiments of static electric induction devices described herein, and
- Figure 12: is a schematic block diagram of an exemplary embodiment of an operation method of static electric induction devices described herein.

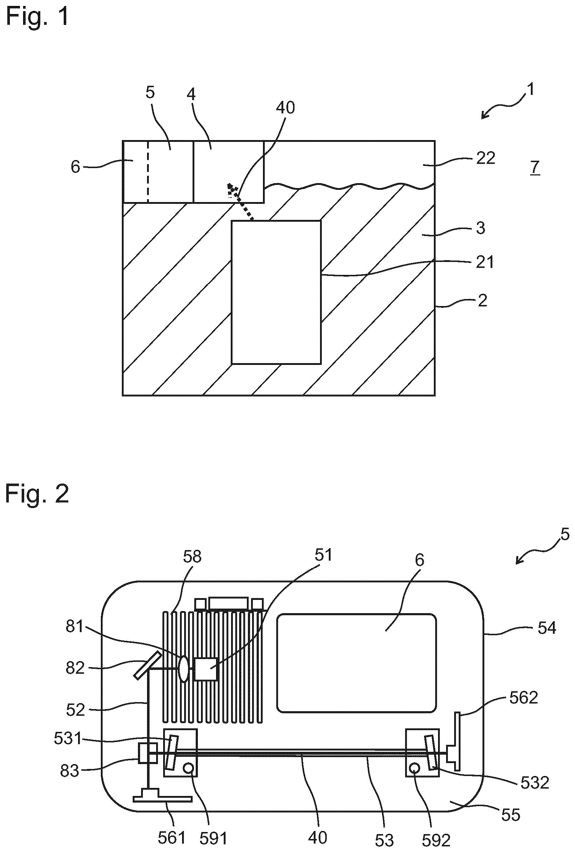

Figure 1 illustrates an exemplary embodiment of a static electric induction device 1. The static electric induction device 1 comprises a tank 2. In the tank 2, there is a static electric induction component 21, for example, a transformer. During operation, the static electric induction component 21 is immersed in an electrically insulating liquid 3 which is, for example, a transformer oil. By means of the electrically insulating liquid 3, the static electric induction component 21 can be cooled. The static electric induction device 1 and especially the tank 2 may be exposed to an exterior atmosphere 7, like air.

Next to the electrically insulating liquid 3, there is a gas extractor 4. The gas extractor 4 may be placed within the tank 2. The gas extractor 4 is configured to extract a gas sample 40 from the electrically insulating liquid 3. For example, the gas extractor 4 is attached to a drain pipe.

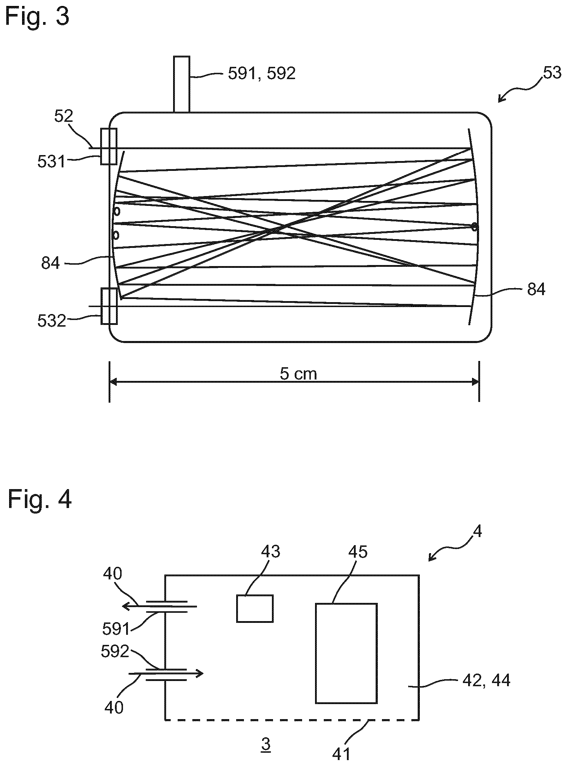

Next to the gas extractor 4, the static electric induction device 1 further comprises a gas analyzer 5. The gas analyzer 5 is configured to receive the gas sample 40 from the gas extractor 4 and to provide concentration data indicative of an oxygen concentration of the gas sample 40. For this purpose, the gas analyzer 5 comprises a laser 51 configured to emit a test radiation 52 which is narrow-band radiation, compare also below the examples of Figures 2 and 4 for more detail. Thus, the concentration data is optical absorption data of the test radiation 52 through the gas sample 40.

As an option, an evaluation unit 6 calculating, for example, a concentration value from the measured optical intensities can also be included in the static electric induction device 1. The evaluation unit 6 may have at least one interface to send the relevant data to an external unit, not shown.

For example, large power transformer devices 1 are generally filled with transformer oil 3 mainly to provide temperature control. It has been learned that the state of health of a transformer 21 can be assessed by determining the state of evolved gases. One possibility to do so is to draw a sample of the transformer oil 3, for example, once a year and analyze the evolved gases in a laboratory by means of gas chromatography. Another possibility is to install evolved gas analyzers directly on the transformer device 1 to continuously monitor the state of evolved gases and thereby the state of the transformer device 1. Continuous monitoring permits actions to be taken to minimize the buildup of evolved gases and thereby extending the useful life of a transformer device 1.

Small amounts of gas can be extracted from the transformer oil 3 in quasi real time by means of a permeable separation membrane 41 of the gas extractor 4. It is thus possible to extract a small quantity of gas, isolate it from the tank 2 and analyze its composition with automated on-line gas chromatography or various methods of spectroscopy.

The extraction of the gas sample 40 from the transformer oil 3 via the permeable separation membrane 41 is a relatively slow process and can take several hours to obtain an adequate quantity of gas for analysis. The repeat cycle time of each analysis, which can be a number of hours, is a strong function of the amount of gas needed to achieve an adequate sensitivity such as to provide a reliable report on the state of health of the transformer device 1.

To avoid the on-line gas chromatography, various techniques of spectroscopy can be used so as to avoid the need for consumables such as carrier and calibration gases and thereby permit long periods of autonomous operation. The optimization of response time of on-line transformer monitoring relates to the combination of time required to extract an adequate quantity of gas for the gas sample 40 and the time required to achieve a sufficient signal quality of, for example, a spectrometer to make a reliable observation.

In principle, there are two approaches for gas sampling:
1) Collecting gas through the permeable separation membrane 41 for a fixed amount of time followed by isolating the gas sample 40 and proceeding with analysis in, for example a batch processing.
2) Collecting gas through the permeable separation membrane 41 into a permanently fixed sealed volume and analyzing time variations of the concentrations of the different species continuously. This is like measuring the gas species in situ after separating the liquid phase of the transformer oil 3 from the gas phase in the sampling volume.

It is to be noted that in the batch processing mode 1), a given amount of gas may be collected in a sealed volume which is isolated from the permeable separation membrane 41 and is analyzed. Several types of oxygen analyzers consume the oxygen as it is being analyzed. This is suitable for a flowing stream of gas containing oxygen but is less desirable for a fixed volume. For the continuous sampling mode 2), consumption of oxygen in the process of analysis will perturb the equilibrium of concentration between dissolved oxygen in the transformer oil 3 and the extracted oxygen in the sealed volume. This is especially undesirable given the slow rate of exchange of the permeable separation membrane 41. This limits the use of oxygen analyzers that perturb the concentration while measuring or consuming oxygen during analysis.

There are accepted threshold concentrations of the various different gas species dissolved in transformer oil 3 below which the operation of the transformer device 21 is in a safe operating regime and above which there is action required such that the concentrations are reduced back to below their safe limits.

Especially the need for oxygen analysis in transformer oil 3 relates to hermetically sealed transformer tanks 2 that are flushed with nitrogen to avoid oxidation of components of the transformer 21 and other components within the tank 2, for example.

For the majority of gas species of interest to be analyzed dissolved in transformer oil 3, infrared spectroscopy may be used. These include a series of hydrocarbons and oxides of carbon. However, oxygen is a symmetric molecule which does not significantly absorb in the infrared spectral range. Since oxygen is weakly paramagnetic, it absorbs very weakly in the 760 nm near-infrared spectral region. The absorption is too weak to be measured by conventional near-infrared spectroscopy. However, single mode tunable diode lasers operating at 760 nm in the near-infrared spectral range may be used for oxygen analysis. These lasers may emit around to 10 mW of optical power distributed over a very narrow wavelength interval such that the power density per unit wavelength interval is much higher than in case of any conventional light source, like light bulbs. This permits the rapid measurement of a small part of the weak oxygen spectrum with a high signal-to-noise ratio. This kind of spectrometric measurement of oxygen concentration can be implemented by means of an absorption cell that is connected in parallel to the absorption cell of an infrared spectrometer for other extracted gas phase species sharing the same extracted gas sample, for example.

In the monitoring of transformer oil 3, the increase in concentration of the various extracted gas phase species provides indications of possible incorrect operating conditions or developing faults of the transformer device 1. It is of great interest to provide rapid awareness of such developments so that prompt actions can be taken to mitigate these conditions. In the batch mode 1) or the continuous monitoring mode 2) mentioned above, the response time to a developing fault is highly dependent on the rate of extraction of gas from the liquid transformer oil 3. In the continuous mode 2), an equilibrium concentration is established after initial startup. As long as the analysis gives the same results, there is no change in the state of the transformer device 1. When the concentrations of some gases changes, there is an indication that a change in transformer operation is occurring. The response time constant of the monitoring system depends on the rate of transmission of the permeable separation membrane 41 of the different gases as well as the spectrometric measurement time. To minimize the latter time delay, the amount of gas required for the analysis should be minimized. But, secondly, a sufficiently large gas sample 40 is needed to obtain a short spectrometric measurement time and the required accuracy. The amount of gas sample required to obtain a valid concentration measurement could be reduced by increasing an optical path length for absorption spectroscopy to provide high spectrometric sensitivity and, hence, minimum measurement time.

For example, a single mode laser has a very narrow line width which could be in the range of about 2 MHz which translates to about 0.00007 cm⁻¹ or 0.000004 nm in the spectral range around 760 nm of particular interest. Such narrow emission lines carry virtually all the power of the emitted laser radiation. For example, a laser emitting just 5 mW of optical power can result in a spectral density of around 1250 W/nm. The availability of relatively low-cost and highly reliable lasers precisely tuned to the spectral domain of oxygen can make it an efficient method of analysis over a wide range of applications and covering a wide range of concentrations down to ppm levels.

Thus, spectral analysis of oxygen can benefit from the more than four orders of magnitude greater specific brightness of a simple single mode diode laser compared with other light sources. For example, an incandescent source for infrared spectroscopy has a luminous power of approximately 15 W covering a wide spectral range resulting in approximately just 1 x 10⁻⁶ W available at one oxygen spectral line while a single mode diode laser source at 760 nm has a luminous power of approximately 0.01 W but fully available for measuring the oxygen spectral line. Since there are many absorption lines for oxygen in the 760 nm region, there is some flexibility in the actual wavelength of the laser, especially as long as it operates in single mode.

In the example as shown in Figure 2, the gas analyzer 5 comprises the laser 51 that emits the narrow-band test radiation 52. It is possible that the laser 51 is mounted on a temperature control component 58, like a TEC. Further optionally, the laser 51 is followed by a collimator lens 81, like a fast-axis collimator. Furthermore, there may be a plane mirror 82 for simplifying adjustment of the optical system.

The test radiation 52 is split into a measurement beam and a reference beam by means of a beam splitter 83, like a 50:50 beam splitter. The measurement beam travels through a test cavity 53 containing the gas sample 40 and finally to a second radiation detector 562. The reference beam travels to a first radiation detector 561. Both radiation detectors 561, 562 may be silicon detectors. These components as well as the optional evaluation unit 6 may be accommodated in an analyzer housing 54. The analyzer housing 54 may be filled with a housing atmosphere 55 which is, for example, dried air or a protective gas. The analyzer housing 54 may be airtight.

To improve accuracy and to reduce noise, radiation sensitive areas of the radiation detectors 561, 562 may fully or nearly fully be illuminated by the test radiation 52. For this purpose, at least one diffusor may optionally be provided, not shown, for example, in front of the respective one of the radiation detectors 561, 562.

For example, the laser 51 is a vertical-cavity surface-emitting laser, VCSEL, or a distributed feedback, DFB, laser. A VCSEL may provide an output power of about 1 mW. A DFB laser may provide an output power typically in the range of 5 mW to 10 mW. The test radiation 52 is preferably carefully collimated to avoid any losses on the way to the radiation detectors 561, 562. To compensate for possible oxygen in the housing atmosphere 55, paths of the measurement beam and the reference beam in the housing atmosphere 55 have preferably the same length.

The test cavity 53 has a radiation entrance window 531 and a radiation exit window 532. Both windows 531, 532 may be aligned obliquely relative to a beam axis of the measurement beam in order to avoid undesired interference or reflections. The windows 531, 532 may close ends of a tube that contains the gas sample 40. Said tube is, for example, of glass or also of a metal and may have an inner diameter of at least 1 mm and/or of at most 5 mm. The windows 531, 532 could be provided with anti-reflective coatings with regard to the test radiation 52. Further, the test cavity 53 has a first gas access 591 and a second gas access 592 so that the gas sample 40 could be led through the tube. Not only circular tubes but also polygonal tubes may be used.

A universal power supply, not shown, may be present that could provide voltages of +/- 15 V and + 5 V, for example. A so-called arbitrary save generator may optionally also be present, not shown. These components may be included in the evaluation unit 6 which may thus not be only for evaluation but also for control of the gas analyzer 5.

According to Figure 2, the measurement beam is led through the test cavity 53 only once. Thus, the test cavity 53 may have a relatively large length of, for example, at least 10 cm and/or of at most 1 m. Contrary to that, in Figure 3 it is shown that a multi-path cell could also be used. In case of the example of Figure 3, the measurement beam runs 20 times through the gas sample 40. This can be achieved, for example, by a set of two or of three curved mirrors 84. Having an exemplary length of 5 cm, thus an overall path length of the measurement beam through the gas sample 40 of around 100 cm can be achieved. Such or similar multi-pass cells can also be used in other examples of the gas analyzer 5.

Hence, the test cavity 53 is not an optical resonator for the test radiation 52. This may also be true in all other embodiments. Thus, adjustment efforts can be reduced and robustness of the optics can be increased. Further, the gas analyzer 5 is free of any gas chromatography stage and may be for determining the concentration of oxygen only.

In Figure 4, the gas extractor 4 is shown in more detail. In addition to the separation membrane 41 next to the electrically insulating liquid 3, the gas extractor 4 optionally comprises a circulation unit 43, like a ventilator or a pump. The circulation unit 43 may be placed in a test volume 42 that contains extracted gas 44 obtained via the separation membrane 41. By means of the circulation unit 43, the gas sample 40 taken from the extracted gas 44 may be led faster through the gas analyzer 5 compared with gas diffusion only.

For example, the test cavity 53 has a small volume content of around 1 ml while the overall test volume 42 could be significantly larger, like having a volume of about 15 ml, for example.

Optionally, the gas extractor 4 may comprise a further analyzation component 45, for example, located in the test volume 42. The further analyzation component 45 may be used for determining the presence of other species than oxygen like H2 and/or carbon compounds. The further analyzation component 45 may be an infrared spectrometer using a broadband light source, like a bulb.

Such a gas extractor 4 could also be used in all other examples of the static electric induction device 1.

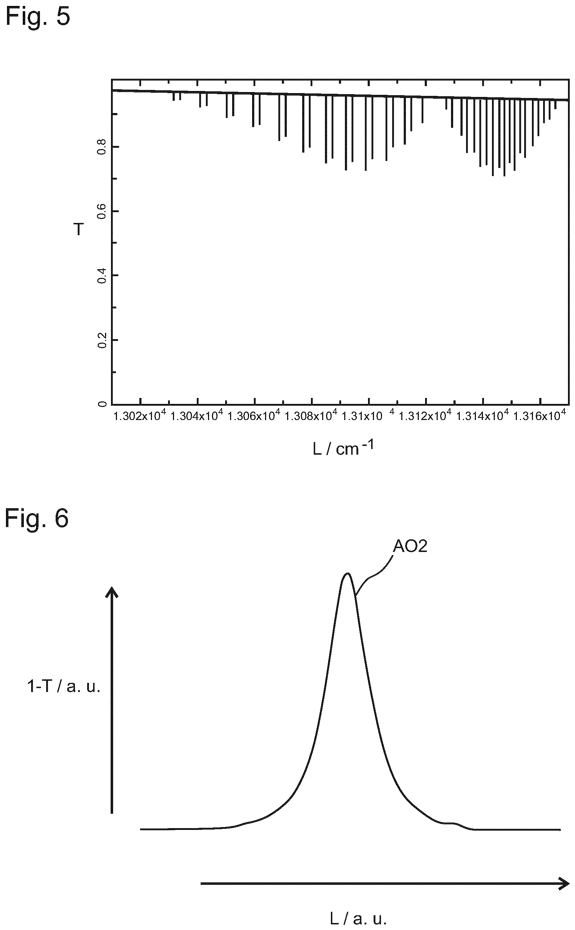

In Figure 5, the transmission T of light through a cell filled with pure oxygen at a pressure of 1 bar and at a path length of 600 cm is shown for the region at a wavelength L of about 760 nm. In Figure 6 the absorption 1-T is shown in more detail for the line at 760,8 nm corresponding to 13144 cm-1. Said absorption line is quite narrowband and can efficiently be scanned by the laser 51.

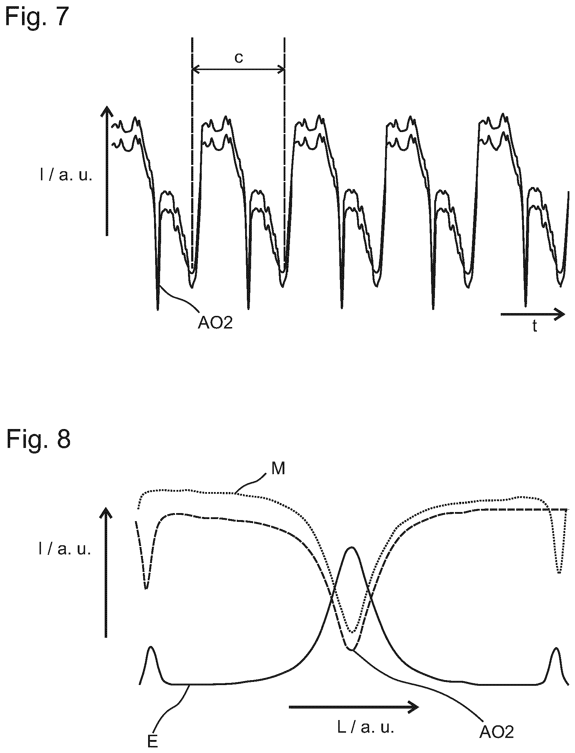

Corresponding scans showing an intensity I over time t are illustrated in Figure 7. The intensity I may correspond to an electric current and/or a voltage measured by means of the radiation detectors 561, 562.

For example, the arbitrary wave generator is set to a triangular waveform at 20 Hz and an offset voltage for a constant drive current term. By adjusting the operating temperature of the laser over a safe temperature range, one or several of the oxygen spectral lines can be visualized. A data sampling rate of detector data is, for example, 4 kHz which provides about 200 samples for one of the triangular wave excursions covering the oxygen absorption line AO2 shown in Figure 6 and leading to the intensity decreases in the scans of Figure 7. For example, 16 bit ADC resolution may be used.

For example, the laser drive current is generated by an arbitrary wave generator set at a symmetrical triangular wave shape or sawtooth wave shape with a nominal frequency of 10 Hz to 50 Hz. An ADC sampling rate was typically set at 4000 samples/s. Furthermore, the laser 51 can be driven with a constant current so that the triangular laser output varies between a high and low non-zero intensity: For example, the constant current to the laser is about 40 mA while the triangular wave amplitude is about 8 mA**.** The triangular wave amplitude determines the wavelength range covered. It normally exceeds the measured spectral line width by a factor of at least three for an atmospheric pressure broadened O₂ full line width.

In Figure 7, a sawtooth wave shape is used consisting of a slow rising ramp voltage followed by a rapid return. Using the measured spectra only for the slow rising ramp provides spectra with repetitive distortion. The data was processed by combining a processed ramp spectrum and adding the ramp in reverse data order centered around the center of the oxygen line and dividing by 2. This symmetrizes the spectrum requiring only a simple baseline offset correction.

In Figure 8, sampling over 1000 is shown corresponding to 50 s observation time. The upper traces are raw coadded spectra and their mirror images which are all asymmetric. Combining, by adding the raw coadded spectra and their mirror images symmetrizes the spectra as shown in lower traces which have been linear baseline corrected.

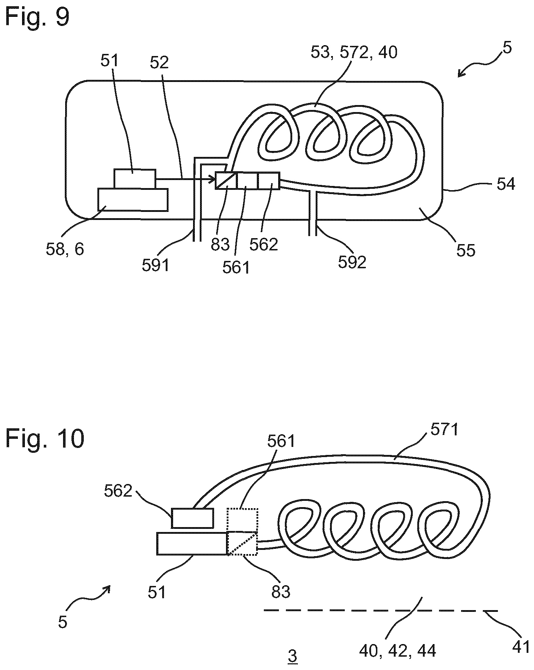

In Figure 9, another example of the gas analyzer 5 is illustrated. In this example, the test cavity 53 is a hollow-core glass fiber 572 to which the gas sample 40 is provided. The radiation detectors 561, 562 may be placed adjacent to one another. As in this configuration all optics requiring adjustment can be placed in close proximity, stability of the optical system can be increased.

Otherwise, the same as to Figures 1 to 8 may also apply to Figure 9, and vice versa.

In the example of Figure 10, a cladding-free optical fiber 571 is used which is placed within the gas sample 40, for example, within the test volume 42. Thus, the gas sample 40 may be sensed by an evanescent field of the test radiation 52. It is possible that the overall optics are arranged in a non-free-beam configuration which could further reduce adjustment efforts and could increase long-term stability.

Otherwise, the same as to Figures 1 to 9 may also apply to Figure 10, and vice versa.

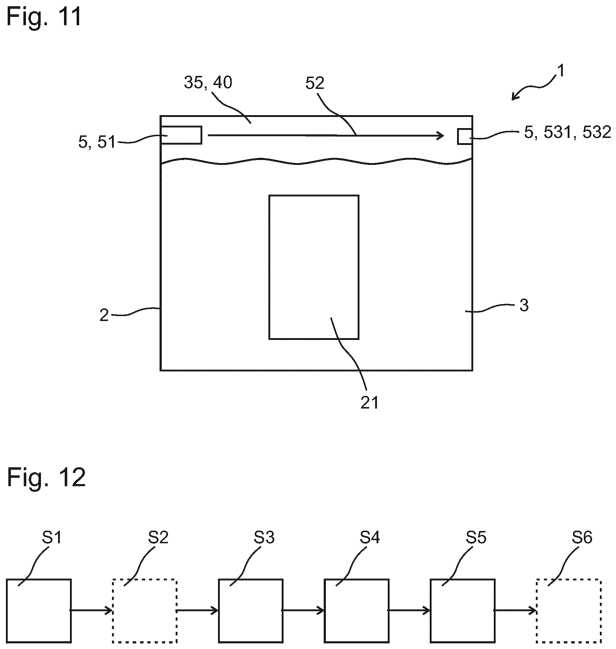

In the example of Figure 11, there is a test space 35 directly atop the electrically insulating liquid 3 in the tank 3. The test space 35 may also be referred to as a head space. The laser 51 of the gas analyzer 5 may directly emit into the test space 35 that comprises the gas sample 40 which outgases from the electrically insulating liquid 3. Also the radiation detector 561, 562 may be directly at or in the test space 35. Accordingly, a membrane of a gas extractor and a test cavity may be omitted.

Optionally, there are further optics, not shown in Figure 11, like a mirror so that the laser 51 and the at least one radiation detector 561, 562 may be next to one another; there could be a measurement beam and a reference beam. Further optionally, the gas analyzer 5 and the at least one radiation detector 561, 562 could be accommodated in a sealed housing having a radiation exit window and a radiation entrance window to let the test radiation into the test space and back, for example, by means of a retroreflector attached to the tank 2. Other than shown in Figure 11, the test space 35 may optionally be located in a pipe system and, thus, not in a main tank volume.

Hence, in this example the oxygen analysis is carried out directly in the head space of the transformer tank 2 such that the gas analyzer 5 is surrounded by evolved gas 40 from the oil 3 so that no sample cell is required, no extractor is required, and the laser 51 may need to operate in a sealed housing having an output window as well as an input window to receive the laser beam within the isolated volume of the analyzer after passing through the evolved gas.

Otherwise, the same as to Figures 1 to 10 may also apply to Figure 11, and vice versa.

In Figure 12, an operation method for the static electric induction device 1 is illustrated.

In a method step S1, the tank 2 is filled with the electrically insulating liquid 3.

In optional method step S2, the static electric induction device 1 is operated.

Then, extracting, by the gas extractor 4, the gas sample 40 from the electrically insulating liquid 3 and transporting the gas sample to the gas analyzer 5 follows as a method step S3.

In method step S4, the test radiation 52 is transmitted by the laser 51 through the gas sample 40.

Then, the gas analyzer 5 provides the concentration data indicative of the oxygen concentration of the gas sample 40.

Optionally, the obtained concentration data can be saved and/or may be sent to an external entity, not shown, for maintenance purposes, for example.

Otherwise, the same as to Figures 1 to 11 may also apply to Figure 12, and vice versa.

The invention described here is not restricted by the description on the basis of the exemplary embodiments. Rather, the invention encompasses any new feature and also any combination of features, which includes in particular any combination of features in the patent claims, even if this feature or this combination itself is not explicitly specified in the patent claims or exemplary embodiments.

### List of Reference Signs

- 1: static electric induction device
- 2: tank
- 21: static electric induction component (transformer)
- 22: interior gas
- 3: electrically insulating liquid
- 35: test space
- 4: gas extractor
- 40: gas sample
- 41: separation membrane
- 42: test volume
- 43: circulation unit
- 44: extracted gas
- 45: further analyzation component
- 5: gas analyzer
- 51: laser
- 52: narrow-band test radiation from the laser
- 53: test cavity
- 531: radiation entrance window
- 532: radiation exit window
- 54: analyzer housing
- 55: housing atmosphere
- 561: first radiation detector
- 562: second radiation detector
- 571: cladding-free optical fiber
- 572: hollow-core glass fiber
- 58: temperature control component
- 591: first gas access
- 592: second gas access
- 6: evaluation unit
- 7: exterior atmosphere (air)
- 81: collimator lens

- 82: plane mirror
- 83: beam splitter
- 84: curved mirror
- AO2: oxygen absorption line
- C: cycle
- E: extracted spectrum
- I: intensity
- L: wavelength in nm or cm⁻¹
- M: measurement data
- S: method step
- t: time
- T: transmission

## Claims

1. A static electric induction device (1) comprising
- a tank (2) configured to be filled with an electrically insulating liquid (3), and
- a gas analyzer (5) configured to provide concentration data indicative of an oxygen concentration of a gas sample (40) obtained from the electrically insulating liquid (3),
wherein
- the gas analyzer (5) comprises a laser (51) configured to emit a test radiation (52) which is narrow-band radiation, and
- the concentration data is optical absorption data of the test radiation (52) through the gas sample (40).

2. The static electric induction device (1) according to the preceding claim,
wherein the test radiation (52) has a wavelength of maximum intensity of at least 760 nm and of at most 765 nm,
wherein the laser (51) is configured for a spectral power density of at least 0.2 kW/nm at the wavelength of maximum intensity.

3. The static electric induction device (1) according to any one of the preceding claims,
wherein the gas analyzer (5) further comprises a test cavity (53) which is configured to be filled with the gas sample (40),
wherein the test cavity (53) is a transmission cell for the test radiation (52).

4. The static electric induction device (1) according to the preceding claim,
wherein for the test radiation (52) the test cavity (53) has a radiation entrance window (531) and a radiation exit window (532) different from the radiation entrance window (531), wherein in the test cavity (53) the test radiation (52) travels a same section in a same direction only once.

5. The static electric induction device (1) according to any one of the two preceding claims,
wherein the test cavity (53) is a multi-pass transmission cell,
wherein the test cavity (53) is of a White cell-type or of a Herriott cell-type.

6. The static electric induction device (1) according to claim 3 or 4,
wherein the test cavity (53) is a hollow-core glass fiber (572).

7. The static electric induction device (1) according to any one of the preceding claims,
further comprising a gas extractor (4) configured to extract the gas sample (40) from the electrically insulating liquid (3),
wherein the gas analyzer (5) is configured to receive the gas sample (40) from the gas extractor (4) prior to providing the concentration data,
wherein the gas analyzer (5) further comprises an analyzer housing (54),
wherein the laser (51) is placed within the analyzer housing (54) and the gas extractor (4) is configured to transport the gas sample (40) into the analyzer housing (54).

8. The static electric induction device (1) according to claims 3 and 7,
wherein the test cavity (53) is located in the analyzer housing (54),
wherein the test radiation (52) travels through a housing atmosphere (55) before entering the test cavity (53), wherein the housing atmosphere (55) comprises oxygen.

9. The static electric induction device (1) according to the preceding claim,
wherein the gas extractor (4) comprises a separation membrane (41) configured to be in direct contact with the electrically insulating liquid (3) and being impermeable for the electrically insulating liquid (3),
wherein the separation membrane (41) is permeable for gases and is configured to be adjacent to a test volume (42) on a side remote from the electrically insulating liquid (3), the test volume (42) is configured to be filled with an extracted gas (44) from the electrically insulating liquid (3) in a diffusion equilibrium with the electrically insulating liquid (3).

10. The static electric induction device (1) according to claim 7 and according to any one of claims 3 to 6, 8 or 9, wherein the gas analyzer (5) further comprises a first radiation detector (561) and a second radiation detector (562) located in the analyzer housing (54),
wherein path lengths of the test radiation (52) outside the test cavity (53) from the laser (51) to the first radiation detector (561) and to the second radiation detector (562) are the same.

11. The static electric induction device (1) according to claim 1 or 2,
wherein the gas analyzer (5) further comprises a cladding-free optical fiber (571) configured to be in direct contact with the gas sample (40),
wherein the test radiation (52) is configured to be led through the cladding-free optical fiber (571) so that an evanescent field of the test radiation (52) reaches into the gas sample (40).

12. The static electric induction device (1) according to claim 1 or 2,
wherein the gas analyzer (5) is placed in a test space (35), the test space (35) is configured to directly adjoin the electrically insulating liquid (3) and is configured to be filled with the gas sample (40),
wherein the concentration data is the optical absorption data of the test radiation (52) through the test space (35).

13. The static electric induction device (1) according to any one of the preceding claims,
wherein the test radiation (52) has a spectral full width at half maximum, FWHM, of at most 0.001 nm,
wherein the electrically insulating liquid (3) is a transformer oil, and
wherein the static electric induction device (1) is a transformer device configured for an electric power of at least 0.1 MVA.

14. The static electric induction device (1) according to any one of the preceding claims,
wherein the laser (51) is configured to vary an output wavelength by at least 0.1 nm and by at most 2 nm,
wherein the laser (51) is temperature-stabilized,
wherein the static electric induction device (1) further comprises an evaluation unit (6) configured to evaluate the concentration data indicative of the oxygen concentration and to output a signal indicative of the oxygen concentration, and
wherein, for providing the concentration data indicative of the oxygen concentration, the gas analyzer (5) is free of an optical resonator and is free of a gas chromatography stage.

15. An operation method for a static electric induction device (1) according to any one of the preceding claims, the operation method comprises:
- filling (S1) the tank (2) with the electrically insulating liquid (3),
- operating (S2) the static electric induction device (1),
- extracting (S3) the gas sample (40) from the electrically insulating liquid (3),
- transmitting (S4), by the laser (51), the test radiation (52) through the gas sample (40), and
- providing (S5), by the gas analyzer (5), the concentration data indicative of the oxygen concentration of the gas sample (40).
